# EUROPEAN PATENT APPLICATION

(11) **EP 0 714 599 A1**
(43) Date of publication of application: **05.06.1996**
(21) Application number: 95118802.8
(22) Date of filing: 29.11.1995
(51) Int. Cl.: A01M 1/20, A61L 9/03

(54) **Tape carrying active agent-containing fine powder**

(30) Priority: 30.11.1994 JP 297402/94
(71) Applicant: SUMITOMO CHEMICAL COMPANY, LIMITED, Osaka-shi, Osaka (JP)
(72) Inventor: Ueda, Minoru, Toyonaka-shi, Osaka-fu (JP); Okada, Kenya, Takarazuka-shi, Hyogo-ken (JP)
(74) Representative: VOSSIUS & PARTNER

(57) **Abstract**

The present invention relates to a tape carrying an active agent-containing fine powder on one surface of a base by a binder and to a method for vaporizing the active agent by heating the tape. In particular, the tape of the present invention has an excellent storage stability for a long time even for any volatile active agent having high vapor pressure because the active agent does not easily volatilize during the storage.

## Description

The present invention relates to a tape for heat vaporization of an active agent such as aromatizing agents, insecticides, bactericides, fungicides or the like.

There can be found several methods for the continuous heat vaporization of an active agent by allowing a tape impregnated with the active agent to pass on a heater element (see, e.g., JP-Y 51-51884/1976, JP-B 55-43727/1980, JP-A 64-80242/1989, JP-Y 4-4538/1992, JP-Y 4-4539/1992, JP-Y 4-4540/1992, JP-B 4-34362/1992).

However, if the above tape is used for the application of a volatile active agent having high vapor pressure at ordinary temperature, such as aromatizing agents, empenthrin, transfluthrin or dimethyl dichlorovinyl phosphate (DDVP), part of the active agent may be lost by volatilization prior to the passage on a heater element for heat vaporization, which makes it difficult to store the tape for a long time. In addition, if an active agent in large quantities is to be carried on a tape, there is no other way than that of using a thick tape, which causes a decrease in the vaporization efficiency of the active agent.

Thus, it is the object of the present invention to overcome the above problems. This object has been achieved by a tape obtainable by incorporating an active agent in a fine powder and applying the active agent-containing fine powder to a tape base.

Thus, the present invention provides a tape comprising an active agent-containing fine powder carried on one surface of a base by a binder.
The present invention provides also a method for heat-vaporizing an active agent, comprising heating the surface not carrying the active agent-containing fine powder of the tape as described above.

The present invention further provides a method for pest control, comprising vaporizing an active agent by heating the surface not carrying the active agent-containing fine powder of the tape as described above, said active agent being selected from the group consisting of insecticides, bactericides and fungicides.

Moreover, the present invention provides a cassette comprising the above tape and an apparatus for receiving the above tape or cassette comprising a heating means for raising the temperature of at least a portion of the tape and optionally a driving means for transporting the tape.

The tape of the present invention has an excellent storage stability for a long time even for any volatile active agent having high vapor pressure because the active agent does not easily volatilize during the storage. The tape of the present invention further has advantages that an active agent in large quantities can be carried thereon and that high heat-vaporization efficiency of an active agent can be attained.

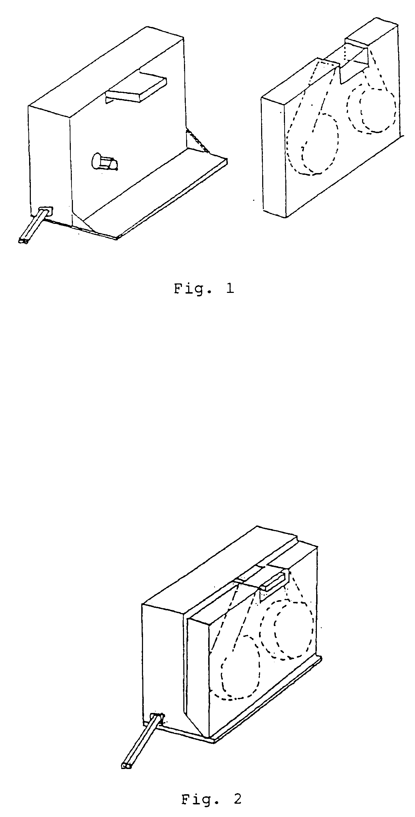

The tape of the present invention is conveniently received in a cartridge and used with a device as shown, for example, in Figures 1 and 2, wherein
Figure 1 is a perspective view showing a device for the heat vaporization of an active agent from an tape of the present invention, and
Figure 2 is a perspective view showing the device of Figure 1 as used.

The tape of the present invention comprises an active agent-containing fine powder carried on one surface of a base by a binder.

The active agent, such as aromatizing agents, insecticides, bactericides and fungicides, to be used in the present invention is not particularly limited, so long as it can be vaporized by heating, even if it is not highly volatile.

Examples of the insecticide are those used for controlling insanitary insects, including pyrethroid compounds such as allethrin, resmethrin, prallethrin, furamethrin, phenothrin, cyphenothrin, permethrin, cypermethrin, empenthrin and transfluthrin, including their effective isomers thereof; organic phosphorous compounds such as dimethyl dichlorovinyl phosphate (DDVP) and fenitrothion; carbamate compounds such as propoxur; and arylazole compounds such as fipronil.

In addition to these insecticides, agricultural and horticultural insecticides for use in greenhouses, including pyrethroids such as fenpropathrin and fluvalinate; organic phosphorous compounds such as chlorpyrifos, diazinon and chorobenzilate, may be used as the active agent of the present invention.

Examples of the fungicide used in the present invention are chlorothalonil, triadimefon, triflumizole, vinclozolin, anilazine and procymidone.

As the aromatizing agent, commercially available aromatizing agents may be used for the present invention.

These active agents may be used alone or in combination with various additives such as efficacy enhancing agents, stabilizers and pigments.

The base used in the present invention is preferably made of a material which can withstand temperature for heat vaporization of an active agent and which can be formed into a film of about 5 to 200 µm in thickness. Examples of the material for the base are laminated paper; woven cloths or non-woven cloths; resins such as polyolefins, polyvinyl chloride, polyvinylidene chloride, polyethylene terephthalate polyfluoroethylene, polyimide and polyamide; resin films of these resins reinforced with grass fibers; aluminum foil; and aluminum-laminated films. Preferred materials for the base are polyethylene terephthalate and polyimide.

Examples of the fine powder used for containing an active agent are powders having high oil-absorbing capacity, usually powders capable of absorbing an oil at an amount by weight which is about 0.2 to 10 times the weight of the powder, e.g., fine powders of mineral carriers such as activated charcoal, activated alumina, acid clay, activated clay, diatomaceous earth, zeolite, attapulgite, silicates bentonite and silica; synthetic reins such as polyvinyl chloride, polyethylene, ethylene-vinyl acetate copolymer and polyacrylic acid; and clathrate compounds such as cyclodextrin. Preferred fine powders are activated charcoal, silicates, silica and polyvinyl chloride powders.

These fine powders usually have a particle size of about 1 to 300 µm, preferably about 5 to 150 µm. The active agent in liquid form is incorporated in a fine powder, as such or as a solution prepared by dissolving in a solvent. The active agent in solid form is incorporated in a fine powder, as a melt or as a solution prepared by dissolving in a solvent.

The amount by weight of active agent to be contained in the fine powder, although it may vary depending upon the kind of fine powder, kind of active agent and the like, is usually about 0.3 to 5 times the weight of the fine powder.

When an active agent-containing fine powder is applied to a base, a binder is used. For example, the active agent-containing fine powder is mixed with a binder-containing liquid, and the mixture was applied to one surface of the base. The binder to be used in the present invention is not particularly limited, so long as the active agent-containing fine powder can be carried on the base. Examples of the binder are ink materials, coating materials and paste materials. The amount of binder is an amount sufficient to ensure that the active agent-containing fine powder can be carried on the base, and the solid content by weight of a binder is usually about 0.05 to 4 times the weight of the active agent-containing fine powder.

The amount of active agent-containing fine powder carried on the base is usually in the range of about 1 to 100 mg/cm².

The width and length of a tape of the present invention may be selected so as to attain the purpose of use, depending upon the kind and amount of active agent, conditions of use, such as time and place for use, and the like. The width of the tape is, for example, in the range of about 6 to 100mm.

When a tape of the present invention is used, an active agent can be vaporized by heating, for example, allowing the surface not carrying the active agent-containing fine powder of the tape to come into contact with a heater element. The heater element is kept at a temperature sufficient to ensure that the active agent can be vaporized, generally about 50° to 300°C.

The running speed of the tape may vary depending upon various factors such as the size of the tape used and the amount of active agent contained, and it is preferably in the range of about 1 to 200 cm/h.

The tape of the present invention can find various applications depending upon the properties of an active agent to be vaporized by heating. For example, in case where the active agent is an insecticide, bactericide or fungicide, the tape of the present invention can be used for pest control, for example, against insects such as mosquitoes, flies and cockroaches, fungi and other pests, by heat vaporization of the active agent therefrom.

### Examples

The present invention will be further illustrated by the following examples which are not to be construed to limit the scope of the present invention. In these examples, the parts are by weight.

### Example 1

Ten parts of an active agent-containing fine powder prepared by incorporating 6.7 parts of jasmine perfume in 3.3 parts of activated charcoal (about 5 to 7 µm particle size) was mixed with 40 parts of an ink material (8 wt% solid content), and the mixture was applied on one surface of a polyimide film (25 µm thickness) to have a thickness of 500 µm in a wet state. After drying, the resulting film was cut in the shape of a tape. The tape thus obtained was stored for one week and then tested by heating on a heater element at 160°C, at which time the aroma of jasmine was detected.

### Example 2

One part of an active agent-containing fine powder prepared by incorporating 0.4 part of jasmine perfume in 0.6 part of powdered polyvinyl chloride (about 120 µm particle size) was mixed with 40 parts of an ink material (8 wt% solid content), and the mixture was applied on one surface of a polyethylene terephthalate film (12 µm thickness) to have a thickness of 500 µm in a wet state. After drying, the resulting film was cut in the shape of a tape. The tape thus obtained was stored for one week and then tested by heating on a heater element at 160°C at which time the aroma of jasmine was detected.

### Example 3

Twelve parts of an active agent-containing fine powder prepared by incorporating 10 parts of d-phenothrin in 2 parts of synthetic hydrated silicon dioxide powder (about 30 µm particle size) was mixed with 7 parts of a coating material (14 wt% solid content), and the mixture was applied on one surface of a polyimide film (25 µm thickness) to have a thickness of 700 µm in a wet state. After drying, the resulting film had d-phenothrin at an amount of 13.8 mg/cm².

The film was cut in the shape of a tape. The tape thus obtained was heated at an area of 4 cm² on a heater element at 280°C and the amount of vaporized active agent was measured to be 43.5 mg (78.8% vaporization rate).

### Example 4

Twelve parts of an active agent-containing fine powder prepared by incorporating 10 parts of permethrin in 2 parts of synthetic hydrated silicon dioxide powder (about 30 µm particle size) was mixed with 7 parts of a coating material (14 wt% solid content), and the mixture was applied on one surface of a polyimide film (25 µm thickness) to have a thickness of 700 µm in a wet state. After drying, the resulting film had permethrin at an amount of 14.1 mg/cm².

The film was cut into the shape of a tape. The tape thus obtained was heated at an area of 4 cm² on a heater element at 280°C and the amount of vaporized active agent was measured to be 45.2 mg (80.1 % vaporization rate).

## Claims

1. A tape comprising an active agent-containing fine powder carried on one surface of a base by a binder.

2. A tape according to claim 1, wherein the active agent is an insecticide.

3. A tape according to claim 1, wherein the active agent is a pyrethroid compound.

4. A tape according to any of claims 1 to 3, wherein the base is made of a material which can withstand temperatures used for heat vaporization of the active agent and which can be formed into a film.

5. A method for heat-vaporizing an active agent, comprising heating the surface not carrying the active agent-containing fine powder of the tape according to any of claims 1 to 4.

6. A method for pest control, comprising vaporizing an active agent by heating the surface not carrying the active agent-containing fine powder of the tape according to any of claims 1 to 4, said active agent being selected from insecticides, bactericides and fungicides.

7. Cassette comprising a tape according to any of claims 1 to 4.

8. Apparatus for receiving a tape according to any of claims 1 to 4 or a cassette according to claim 7 comprising a heating means for raising the temperature of at least a portion of the tape.

9. Apparatus according to claim 8 further comprising a driving means for transporting the tape.
